(19) Europäisches Patentamt
European Patent Office
Office européen des brevets

(11) Veröffentlichungsnummer: **0 088 325**
**B1**

(12) **EUROPÄISCHE PATENTSCHRIFT**

(45) Veröffentlichungstag der Patentschrift: **11.09.85**

(51) Int. Cl.⁴: **C 07 C 131/00**, C 07 D 307/66, C 07 D 309/30, A 01 N 37/46, A 01 N 43/08, A 01 N 43/16

(21) Anmeldenummer: **83101935.1**

(22) Anmeldetag: **28.02.83**

(54) **Oximinoessigsäureamide, ihre Herstellung und ihre Verwendung zur Bekämpfung von Fungi und Mittel dafür.**

(30) Priorität: **09.03.82 DE 3208329**

(43) Veröffentlichungstag der Anmeldung: **14.09.83 Patentblatt 83/37**

(45) Bekanntmachung des Hinweises auf die Patenterteilung: **11.09.85 Patentblatt 85/37**

(84) Benannte Vertragsstaaten: **AT BE CH DE FR GB IT LI NL SE**

(56) Entgegenhaltungen: **DE - A - 2 837 863**

(73) Patentinhaber: **BASF Aktiengesellschaft, Carl-Bosch-Strasse 38, D-6700 Ludwigshafen (DE)**

(72) Erfinder: **Graf, Hermann, Dr., Ginsterstrasse 15, D-6704 Mutterstadt (DE)**
Erfinder: **Zeeh, Bernd, Dr., Thorwaldsenstrasse 5, D-6700 Ludwigshafen (DE)**
Erfinder: **Schaefer, Peter, Dr., Im Buegen 16, D-6719 Kirchheim (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 088 325 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Oximinoessigsäureamide, Verfahren zu deren Herstellung, Fungizide, die diese Verbindungen als Wirkstoffe enthalten, sowie Verfahren zur Bekämpfung von Pilzen.

Oximinoessigsäurederivate sind bereits als Acylierungsmittel bei der Herstellung von Antibiotica bekannt (DE-OS 2 223 287). Weiter ist bekannt, N-Trichlormethylthiophthalimid und N-Trichlormethylthiotetrahydrophthalimid als Fungizide in der Landwirtschaft und im Obst- und Gartenbau einzusetzen (vgl. z. B. Wegler, »Chemie der Pflanzenschutz- und Schädlingsbekämpfungsmittel«, Bd. 2, S. 109 ff. und Bd. 4, Seite 191 ff., Springer Verlag, Berlin/Heidelberg/New York, 1970 bzw. 1977). Die genannten Mittel sind jedoch nach erfolgter Infektion nicht verwendbar und ihre Wirkung genügt bei niedrigen Aufwandmengen nicht den Anforderungen der Praxis.

Es wurde nun gefunden, daß Oximinoessigsäureamide der Formel I

$$R^1-NR^2-CO-CH=N-O-R^3 \qquad (I),$$

in der

$R^1$ 2-Methyl-1-naphthyl, 2,6-Dimethyl-1-cyclohexyl oder den Rest

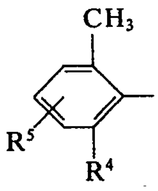

bedeutet, worin $R^4$ Wasserstoff, Methyl, Ethyl oder Chlor und $R^5$ Wasserstoff, Methyl, Phenyl, Chlor oder Brom ist,

$R^2$ den alpha-Butyro-gamma-lactonyl-, alpha-Valero-gamma-lactonyl- oder 1-Butin-3-yl-Rest oder die Gruppe $-CH(CH_3)-R^6$ bedeutet, worin $R^6$ Methoxycarbonyl oder 1,1-Dialkoxymethyl ist, und

$R^3$ einen gegebenenfalls halogensubstituierten $C_{1-6}$-Alkylrest,
einen gegebenenfalls halogensubstituierten Allylrest,
einen gegebenenfalls halogensubstituierten Benzylrest oder einen Propargylrest darstellt,

eine gute fungizide Wirksamkeit besitzen und leicht herstellbar sind.

In der Formel I ist $R^1$ vorzugsweise ein 2,6-Dimethylphenyl- oder 2-Methyl-1-naphthylrest, $R^2$ der alpha-Butyro-gamma-lactonyl- oder der $-CH(CH_3)-CO-OCH_3$-Rest und $R^3$ ein gegebenenfalls chlorsubstituierter Methyl-, Ethyl-, Allyl- oder Benzylrest.

Die Oximinoessigsäureamid der Formel I besitzen im Rest $R^2$ ein Asymmetriezentrum. Durch die eingeschränkte Drehbarkeit der C—N-Bindung im Aminteil können sich bei Raumtemperatur stabile Rotamere (Atropisomere) bilden, zusätzlich besteht im Oximteil die Möglichkeit der syn-anti-Stereoisometrie. Die reinen Enantiomeren bzw. Diastereomere können nach üblichen Methoden erhalten werden. Falls $R^1$ einen 2,6-Dimethyl-1-cyclohexylrest darstellt, können all-trans, all-cis oder cis-trans-Isomere vorliegen. Die Erfindung schließt die Isomeren und deren Gemische ein.

Die neuen Oximinoessigsäureamide der Formel lassen sich herstellen, indem man

a) ein Amin der Formel II

$$R^1-NH-R \qquad (II),$$

worin $R^1$ und $R^2$ die angegebene Bedeutung haben, mit einer Verbindung der Formel III

$$R^3-O-N=CH-CO-X \qquad (III),$$

worin $R^3$ die angegebene Bedeutung hat und X ein aktiver Säurerest ist, in Gegenwart einer Base oder

b) ein Amin der Formel II mit einer Säure der Formel IV

$$R^3-O-N=CH-COOH \qquad (IV),$$

worin $R^3$ die angegebene Bedeutung hat, in Gegenwart eines Säurechlorids und einer Base oder

c) eine Verbindung der Formel V

$$R^1-NH-CO-CH=N-O-R^3 \qquad (V),$$

worin $R^1$ und $R^3$ die angegebene Bedeutung besitzen, mit einer Verbindung der Formel

$R^2-Z$ (VI);

worin $R^2$ die angegebene Bedeutung und Z eine nucleofuge Gruppe darstellt, in Gegenwart einer Base umsetzt.

Für die Umsetzung a), die bevorzugt ist, kommen als Verbindungen der Formel III die entsprechende Oximinoessigsäurechloride oder -bromide, gemischte Säureanhydride, welche einen O-substituierten Oximinoessigsäurerest enthalten, entsprechende O-substituierte Oximinoessigsäureimidazolide oder -triazolide in Betracht. Die Reaktion a) wird zweckmäßigerweise in einem Lösungs- oder Verdünnungsmittel unter Zusatz einer Base und eines Reaktionsbeschleunigers durchgeführt.

Lösungs- oder Verdünnungsmittel sind z. B. aliphatische oder aromatische Kohlenwasserstoffe — wie Pentan, Cyclohexan, Petrolether, Benzol, Toluol oder Xylol-; Halogenkohlenwasserstoffe — wie Methylenchlorid, 1,2-Dichlorethan oder Chlorbenzol-; Ether — wie Diethylether, 1,2-Dimethoxyethan, Tetrahydrofuran oder 1,4-Dioxan-; Ester — wie Essigsäureethylester-, ferner Acetonitril, Dimethylsulfoxid, N,N-Dimethylformamid oder Acetanhydrid.

Als anorganische oder organische Basen kommen z. B. Alkali- oder Erdalkalicarbonate — wie Natrium- oder Kaliumhydrogencarbonat, Natrium- oder Kaliumcarbonat, Calcium- oder Bariumcarbonat-; Amine — wie Triethylamin, N,N-Dimethylanilin, N-Methylpiperidin oder Pyridin — in Betracht.

Als Reaktionsbeschleuniger eignen sich z. B. Metallhalogenide — wie Natriumbromid oder Kaliumiodid-, Azole — wie Imidazol oder 1,2,4-Triazol-Pyridine — wie 4-Dimethylaminopyridin- oder Dimethylformamid.

Anstelle der Säurederivate III kann man gemäß Verfahren b) auch die entsprechenden freien Säuren zusammen mit organischen oder anorganischen Säurehalogeniden oder Aktivierungsreagentien verwenden.

Geeignete anorganische oder organische Säurehalogenide sind z. B. Thionylchlorid, -bromid, Phosphortrichlorid, -bromid, Phosphoroxychlorid, -bromid, Phosphorpentachlorid, -bromid, Oxalylchlorid, -bromid, Acetylchlorid oder p-Toluolsulfonsäurechlorid.

Als Aktivierungsreagentien kommen z. B. 1,1-Dichlormethyl-methyl-ether, Dicyclohexylcarbodiimid, N-Ethyl-N'-(3-dimethylaminopropyl)-carbodiimid, Carbonyldiimidazol, Thionyldiimidazol, Sulfuryldiimidazol, 1-Methyl-2-chlorpyridinium-iodid oder — tetrafluoroborat sowie 4,6-Diphenylthieno[3,4-d][1,3]-dioxol-2-on-5,5-dioxid in Betracht.

Als Basen und Lösungs- bzw. Verdünnungsmittel eignen sich für das Verfahren b) dieselben wie für a).

Für das Verfahren c) kommen als Lösungs- oder Verdünnungsmittel in Betracht: Ether — wie Diethylether, Diisopropylether, Tetrahydrofuran, 1,4-Dioxan oder 1,2-Dimethoxy-ethan-; außerdem N,N-Dimethylformamid, Dimethylsulfoxid oder Sulfolan.

Als Basen für das Verfaren c) seinen genannt: Alkali- oder Erdalkali-hydride — wie Lithiumhydrid, Natriumhydrid, Kaliumhydrid oder Calciumhydrid — Alkaliamide — wie Natrium- oder Kaliumamid sowie Lithiumdiisopropylamid oder -tetramethylpiperid. Zudem kann unter Phasentransferbedingungen gearbeitet werden mit Solvens-Base-Systemen wie Methylenchlorid/wäßriges Natrium- oder Kaliumhydroxid, 1,2-Dichlorethan/wäßriges Natrium- oder Kaliumhydroxid, oder Chlorbenzol/wäßriges Natrium- oder Kaliumhydroxid. Als Katalysatoren hierfür dienen Ammonium, Phosphonium- oder Sulfoniumsalze; speziell seien genannt: Tri-n-butyl-ammoniumhydroxid, -hydrogensulfat oder -bromid; Triethylbenzylammonium-chlorid, Methyl-tri-octyl-ammonium-chlorid oder Methyltriphenylphosphoniumbromid.

Die Reaktion wird bei Temperaturen zwischen −50 und +100°C, vorzugsweise zwischen −10 und +40°C vorgenommen.

Die für die oben genannten Verfahren a—c benötigten Ausgangsstoffe lassen sich wie folgt herstellen:

Die Verbindungen III erhält man durch Umsetzung der entsprechenden freien Säuren mit einem aktivierten Säurederivat — wie Oxalylchlorid — in Anwesenheit von Dimethylformamid in Toluol bei 40°C. Die freien Säuren IV erhält man beispielsweise durch Umsetzen von Glyoxylsäure mit O-substituierten Acetonoximen in Cyclohexanon bei 80°C in Gegenwart einer geringen Menge Salzsäure.

Die Verbindungen V lassen sich aus den entsprechenden Isonitrosoacetamiden, deren Herstellung analog der bekannten Herstellung von Isonitrosoacetaniliden erfolgt, darstellen, indem man diese mit einer Verbindung der Formel $R^3Z$ in Gegenwart einer Base O-alkyliert, wobei $R^3$ die angegebene Bedeutung besitzt und Z für eine nucleofuge Gruppe wie Chlor, Brom oder Jod, p-Toluolsulfonyl, p-Brombenzolsulfonyl, Methansulfonyl, Trifluormethansulfonyl, p-Nitrobenzolsulfonyl, Nonabutansulfonyl oder Trifluorethansulfonyl steht.

Die nachfolgenden Beispiele dienen zur Erläuterung der Erfindung. Wenn nicht anders vermerkt, ist bei Nennung eines Wirkstoffs stets das Gemisch der möglichen Stereoisomeren gemeint.

## Beispiel 1

### A. Herstellung des Ausgangsmaterials

#### a) O-Methyl-oximinoessigsäure

In 150 ml Ethanol wurden 8,35 g (100 mmol) O-Methylhydroxylammoniumchlorid, 9,21 g (100 mmol) Glyoxylsäurehydrat sowie 8,0 g Kaliumacetat vorgelegt und auf 60°C erwärmt. Durch Zugabe von Kaliumcarbonat stellte man auf pH 4,5 ein und rührte 24 h bei 60°C. Danach ließ man abkühlen, stellte mit 1 N NaOH auf pH 9—10, wusch dreimal mit je 100 ml Methylenchlorid, trennte die wäßrige Phase ab und stellte sie mit Salzsäure auf pH 2—3 ein. Die so erhaltene Lösung wird längere Zeit mit Methylenchlorid perforiert, wobei der pH der Lösung auf 2—3 gehalten wurde. Nach Abtrennen, Abdestillieren und Trocknen blieben 6,26 g (72%) festes Material zurück, Fp = 50—53°C.

#### b) O-Methyl-oximinoessigsäurechlorid

5,2 g (60 mmol) O-Methyl-oximinoessigsäure und 2 Tropfen trockenes N,N-Dimethylformamid wurden in 50 ml trockenem Toluol vorgelegt und 8,5 ml (12,7 g; 100 mmol) Oxalylchlorid langsam zugetropft; nach Abklingen der Gasentwicklung wurde 30 min auf 40°C erwärmt. Bei Destillation ging das Säurechlorid zusammen mit Toluol bei 100—110°C über.

### B. Herstellung des Endprodukts

#### N-(1-Methoxycarbonyl-1-ethyl)-N-(2,6-dimethylphenyl)-(O-methyl)-oximinoacetamid

10,4 g (50 mmol) N-(2,6-Dimethylphenyl)-alaninmethylester wurden zusammen mit einer Spatelspitze 4-Dimethylamino-pyridin in 250 ml trockenem Toluol vorgelegt. Unter Kühlung tropfte man 50 mmol O-Methyl-oximinoessigsäurechlorid, gelöst in Toluol, zu. Anschließend wurde 3 h bei 70°C nachgerührt, wobei man einen schwachen Stickstoffstrom durch die Lösung perlen ließ. Nach Abkühlen wurde die Lösung dreimal mit je 100 ml Wasser gewaschen, getrocknet und eingeengt. Das anfallende Öl (12,0 g; 82%) wurde säulenchromatographiert, was zu 9,2 g zähflüssiger Reinsubstanz führte.

IR($cm^{-1}$): 1455 (breit), 1385 (breit), 1275, 1265, 1200 (breit), 1180, 1125, 1065, 1045, 975, 780

## Beispiel 2

### A. Herstellung des Ausgangsmaterials

#### a) O-Ethyl-oximinoessigsäure

27,3 g (300 mmol) Glyoxylsäurehydrat und 30,3 g (300 mmol) Acetonoxim-O-ethylether wurden in 200 ml 1,2-Dimethoxyethan bis zum leichten Sieden erwärmt. Unter hohem Rücklaufverhältnis wurde über eine kurze Kolonne das entstehende Aceton/Wasser/1,2-Dimethoxyethan-Azeotrop (Übergang 60—79°C) fortlaufend abdestilliert. Als die Destillattemperatur 82°C überstieg, wurde abgebrochen, abgekühlt und das Lösungsmittel entfernt. Das zurückbleibende Öl erstarrte beim Erkalten; Ausbeute 31,8 g (91%). Nach Anreiben mit Ether ergaben sich farblose Schüppchen, Fp = 74—77°C.

#### b) O-Ethyl-oximinoessigsäurechlorid

7,02 g (60 mmol) O-Ethyl-oximinoessigsäure und zwei Tropfen trockenes N,N-Dimethylformamid wurden in 50 ml trockenem Toluol gelöst und langsam 8,7 ml (12,7 g;100 mmol) Oxalylchlorid hinzugefügt. Nach Abklingen der Gasentwicklung wurde 30 min auf 40°C erhitzt und anschließend Solvens sowie überschüssiges Oxalylchlorid vorsichtig im Vakuum abgezogen. Man erhielt 9,30 g eines blaßbraunen Pulvers, das noch Spuren von N,N-Dimethylformamid und Toluol enthielt.

H—NMR ($CDCl_3$): $\delta$ = 1,40 (t, $CH_3$ J = 7 Hz), 4,45 (g, $CH_2$), 7,65 (s, CH) ppm.

B. Herstellung des Endproduktes

N-alpha-Butyro-gamma-lactonyl)-N-(2,6-dimethyl-phenyl)-(O-ethyl)-oximinoacetamid

22,6 g (110 mmol) N-(alpha-Butyro-gamma-lactonyl)-2,6-dimethylanilin und eine Spatelspitze 4-Dimethylaminopyridin wurden in 250 ml trockenem Toluol in Lösung gebracht und mit 110 mmol O-Ethyloximinoessigsäurechlorid, gelöst in Toluol, versetzt. Nach Beendigung der Wärmeentwicklung wurde 4 h bei 70°C nachgerührt, wobei ein schwacher Stickstoffstrom durch die Lösung geleitet wurde. Danach ließ man abkühlen, wusch dreimal mit je 100 ml Wasser, trocknete und engte ein, was zu 25,1 g (75%) eines in der Kälte erstarrenden Öls führte. Nach Verreiben mit Diethylether schmolz die Substanz bei 133—135°C.

Nach den genannten Methoden wurde folgende O-subst. Oximinoessigsäureamide der Formel I dargestellt bzw. lassen sich darstellen:

Tabelle

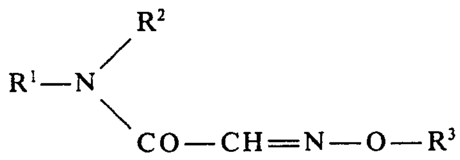

(I)

| Beispiel | $R^1$ | $R^2$ | $R^3$ | phys. Daten<br>Schmp. (°C)/Brech.-Index/<br>IR-Banden ($cm^{-1}$; finger-print) |
|---|---|---|---|---|
| 3 | 2,6-$(CH_3)_2$-$C_6H_3$ | $CH(CH_3)-COOCH_3$ | $C_2H_5$ | Öl; IR: 1412, 1384, 1225, 1180, 1169 (stark), 1048, 1033, 973, 776; |
| 4 | 2,6-$(CH_3)_2$-$C_6H_3$ | $CH(CH_3)-COOCH_3$ | Allyl | Öl; n [illegible] = 1,5275 |
| 5 | 2,6-$(CH_3)_2$-$C_6H_3$ | $CH(CH_3)-COOCH_3$ | 1-Cl-3-Allyl | |
| 6 | 2,6-$(CH_3)_2$-$C_6H_3$ | $CH(CH_3)-COOCH_3$ | 2-Cl-3-Allyl | |
| 7 | 2,6-$(CH_3)_2$-$C_6H_3$ | $CH(CH_3)-COOCH_3$ | Benzyl | 76—78; 1450, 1375, 1365 (breit), 1200, 1175, 1125, 1020 (breit), 985, 970, 695; |
| 8 | 2,6-$(CH_3)_2$-$C_6H_3$ | $CH(CH_3)-COOCH_3$ | 4-Cl-Benzyl | |
| 9 | 2,6-$(CH_3)_2$-$C_6H_3$ | $CH(CH_3)-COOCH_3$ | 2,4-$Cl_2$-Benzyl | |
| 10 | 2,6-$(CH_3)_2$-$C_6H_3$ | $CH(CH_3)-COOCH_3$ | 2,6-$Cl_2$-Benzyl | |
| 11 | 2,6-$(CH_3)_2$-$C_6H_3$ | $CH(CH_3)-COOCH_3$ | Propargyl | |
| 12 | 2,6-$(CH_3)_2$-$C_6H_3$ | alpha-Butyro-gamma-lactonyl | $CH_3$ | |
| 13 | 2,6-$(CH_3)_2$-$C_6H_3$ | alpha-Butyro-gamma-lactonyl | $C_2H_5$ | 133—135 |
| 14 | 2,6-$(CH_3)_2$-$C_6H_3$ | alpha-Butyro-gamma-lactonyl | n-$C_3H_7$ | |
| 15 | 2,6-$(CH_3)_2$-$C_6H_3$ | alpha-Butyro-gamma-lactonyl | Allyl | 88—89 |

Tabelle (Fortsetzung)

| Beispiel | $R^1$ | $R^2$ | $R^3$ | phys. Daten<br>Schmp. (°C)/Brech.-Index/<br>IR-Banden ($cm^{-1}$; finger-print) |
|---|---|---|---|---|
| 16 | 2,6-$(CH_3)_2$-$C_6H_3$ | alpha-Butyro-gamma-lactonyl | 1-Cl-3-Allyl | Öl; IR: 1474, 1413 (breit), 1264, 1223, 1171, 1030, 1015, 1002, 969 (breit) |
| 17 | 2,6-$(CH_3)_2$-$C_6H_3$ | alpha-Butyro-gamma-lactonyl | 2-Cl-3-Allyl | |
| 18 | 2,6-$(CH_3)_2$-$C_6H_3$ | alpha-Butyro-gamma-lactonyl | Benzyl | 46—50 |
| 19 | 2,6-$(CH_3)_2$-$C_6H_3$ | alpha-Butyro-gamma-lactonyl | 4-Cl-Benzyl | Öl; IR: 1492, 1409, 1223, 1171, 1030, 1015, 1002, 969 (breit) |
| 20 | 2,6-$(CH_3)_2$-$C_6H_3$ | alpha-Butyro-gamma-lactonyl | 2,4-$Cl_2$-Benzyl | |
| 21 | 2,6-$(CH_3)_2$-$C_6H_3$ | alpha-Butyro-gamma-lactonyl | 2,6-$Cl_2$-Benzyl | 170—173 |
| 22 | 2-$CH_3$-1-naphthyl | $CH(CH_3)-COOCH_3$ | $CH_3$ | |
| 23 | 2-$CH_3$-1-naphthyl | $CH(CH_3)-COOCH_3$ | $C_2H_5$ | Öl; IR: 1390, 1378, 1276, 1203, 1180, 1141, 1029, 977 (breit), 820, 790 |
| 24 | 2-$CH_3$-1-naphthyl | $CH(CH_3)-COOCH_3$ | n-$C_3H_7$ | |
| 25 | 2-$CH_3$-1-naphthyl | $CH(CH_3)-COOCH_3$ | Allyl | Öl; IR: 1390, 1378, 1276, 1203 (stark), 1180, 1141, 1029, 977 (breit), 945, 820, 791 |
| 26 | 2-$CH_3$-1-naphthyl | $CH(CH_3)-COOCH_3$ | 1-Cl-3-Allyl | Öl; |
| 27 | 2-$CH_3$-1-naphthyl | $CH(CH_3)-COOCH_3$ | 2-Cl-3-Allyl | Öl; IR: 1455, 1391 (stark), 1277, 1247, 1202 (stark), 1178, 1065, 819 |
| 28 | 2-$CH_3$-1-naphthyl | $CH(CH_3)-COOCH_3$ | Benzyl | Öl; IR: 1454, 1390, 1273, 1245, 1202, 1180, 965, 819, 791, 750 |
| 29 | 2-$CH_3$-1-naphthyl | $CH(CH_3)-COOCH_3$ | 4-Cl-Benzyl | Öl; IR: 1408, 1391 (Doppelbande, breit), 1274, 1203, 1014, 962, 817 |
| 30 | 2-$CH_3$-1-naphthyl | $CH(CH_3)-COOCH_3$ | 2,4-$Cl_2$-Benzyl | |

Tabelle (Fortsetzung)

| Beispiel | $R^1$ | $R^2$ | $R^3$ | phys. Daten<br>Schmp. (°C)/Brech.-Index/<br>IR-Banden ($cm^{-1}$; finger-print) |
|---|---|---|---|---|
| 31 | 2-$CH_3$-1-naphthyl | $CH(CH_3)-COOCH_3$ | 2,6-$Cl_2$-Benzyl | 92—95; IR: 1438, 1390 (breit), 1270 (breit), 1200, 1180, 995, 960, 785, 760 |
| 32 | 2-$CH_3$-1-naphthyl | alpha-Butyro-gamma-lactonyl | $CH_3$ | Öl; IR: 1413, 1382, 1267, 1175, 1175, 1050, 1028, 969, 818 |
| 33 | 2-$CH_3$-1-naphthyl | alpha-Butyro-gamma-lactonyl | $C_2H_5$ | Öl; IR: 1504, 1410, 1368, 1264, 1170 (breit), 1045 (stark, breit), 1030, 970, 955, 815, 780 |
| 34 | 2-$CH_3$-1-naphthyl | alpha-Butyro-gamma-lactonyl | n-$C_3H_7$ | |
| 35 | 2-$CH_3$-1-naphthyl | alpha-Butyro-gamma-lactonyl | Allyl | Öl; IR: 1509, 1415, 1378, 1173, 1024 (stark), 985, 965, 814, 782 |
| 36 | 2-$CH_3$-1-naphthyl | alpha-Butyro-gamma-lactonyl | 1-Cl-3-Allyl | |
| 37 | 2-$CH_3$-1-naphthyl | alpha-Butyro-gamma-lactonyl | 2-Cl-3-Allyl | |
| 38 | 2-$CH_3$-1-naphthyl | alpha-Butyro-gamma-lactonyl | Benzyl | 85—90 |
| 39 | 2-$CH_3$-1-naphthyl | alpha-Butyro-gamma-lactonyl | 4-Cl-Benzyl | Öl; IR: 1492, 1411, 1380, 1174, 1026, 1015, 962, 815 |
| 40 | 2-$CH_3$-1-naphthyl | alpha-Butyro-gamma-lactonyl | 2,4-$Cl_2$-Benzyl | |
| 41 | 2-$CH_3$-1-naphthyl | alpha-Butyro-gamma-lactonyl | 2,6-$Cl_2$-Benzyl | 85—90; IR: 1582, 1565, 1436, 1412, 1385, 1181, 1035, 1026, 960 (stark), 820 |
| 42 | 2,6-$(CH_3)_2$-$C_6H_3$ | Butinyl | $C_2H_5$ | |
| 43 | 2,6-$(CH_3)_2$-$C_6H_3$ | alpha-Valero-gamma-lactonyl | $C_2H_5$ | |
| 44 | 2,6-$(CH_3)_2$-$C_6H_3$ | $CH(Me)(OMe)_2$ | $C_2H_5$ | |
| 45 | cis-2,6-Dimethylcyclohexyl | $CH(Me)CO_2Me$ | $C_2H_5$ | Öl; IR: 1439 (stark, breit), 1382, 1245, 1222 (breit), 1092, 1048 (stark), 958 (breit) |

Tabelle (Fortsetzung)

| Beispiel | $R^1$ | $R^2$ | $R^3$ | phys. Daten Schmp. (°C)/Brech.-Index/ IR-Banden ($cm^{-1}$; finger-print) |
|---|---|---|---|---|
| 46 | 2-$CH_3$-$C_6H_4$ | $CH(Me)CO_2Me$ | $C_2H_5$ | |
| 47 | 2-Cl-6-$CH_3$-$C_6H_3$ | $CH(Me)CO_2Me$ | $C_2H_5$ | |
| 48 | 4-Br-2,6-$(CH_3)_2$-$C_6H_2$ | $CH(Me)CO_2Me$ | $C_2H_5$ | |
| 49 | 4-$C_6H_4$-2,6-$(CH_3)_2$-$C_6H_2$ | $CH(Me)CO_2Me$ | $C_2H_5$ | |

Die neuen Verbindungen sind insbesondere geeignet zur Bekämpfung folgender Pflanzenkrankheiten:

Phytophthora infestens an Tomaten und Kartoffeln,
Phytophthora parasitica an Erdbeeren,
Phytophthora cactorum an Äpfeln,
Pseudoperonospora cubensis an Gurken,
Pseudoperonospora humuli an Hopfen,
Peronospora destructor an Zwiebeln,
Peronospora sparsa an Rosen,
Peronospora tabacina an Tabak,
Plasmopara viticola an Reben,
Plasmopara halstedii an Sonnenblumen,
Pythium ultimum an Erbsenkeimlingen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollten in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen) Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle( z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methylalpha-pyrrolidon und erhält eine Lösung, die zur Anwendung in Form kleinster Tropfen geeignet ist.

II. 10 Gewichtsteile der Verbindung des Beispiels 2 werden in einer Mischung gelöst, die aus 90 Gewichtsteilen Xylol, 6 Gewichtsteilen des Anlagerungsproduktes von 8 bis 10 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

III. 20 Gewichtsteile der Verbindung des Beispiels 4 werden in einer Mischung gelöst, die aus 60 Gewichtsteilen Cyclohexanon, 30 Gewichtsteilen Isobutanol, 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile der Verbindung des Beispiels 7 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 80 Gewichtsteile der Verbindung des Beispiels 13 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 15 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 32 werden mit 10 Teilen Natriumsalz eines Phenolsulfonsäure-harnstoff-formaldehyd-Kondensats, 2 Keilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

IX. 20 Teile der Verbindung des Beispiels 2 werden mit 2 Teilen Calciumsalz der Dodecylbenzylsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat),
und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;

Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;

heterocyclische Substanzen, wie
N-(1,1,2,2-Tetrachlorethylthio)-tetrahydrophthalimid,
N-Trichlormethylthio-tetrahydrophthalimid,
2-Heptadecyl-2-imidazolin-acetat
2,4-Dichlor-6-(o-chloranilino)-s-triazin
O,O-Diäthyl-phthalimidophonothioat
5-Amino-1-(bis-(dimethylamino)-phosphinyl)-3-phenyl-1,2,4-triazol)
2,3-Dicyano-1,4-dithioanthrachinon
2-Thio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4-4-dioxid
2,3-Dihydro-5-carboxanilido-6-methyl-1,4-oxathiin
2-(Furyl-(2)-benzimidazol
Piperazin-1,4-diylbis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis-(3-äthoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyäthyl)-glutarimid
Hexachlorbenzol
N-Dichlorfluormethylthio-N,N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichloräthan
2,6-Dimethyl-N-tridecyl-morpholin bzw. dessen Salze
2,6-Dimethyl-N-cyclododecyl-morpholin bzw. dessen Salze
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin,
D,L-N-(2,6-Dimethylphenyl)-N-(phenylacetyl)-alaninmethylester,
1-[2-(2,4-Dichlorphenyl)-4-n-propyl-1,3-dioxolan-2-yl-methyl]-1H-1,2,4-triazol.

Die folgenden Beispiele belegen die biologische Mischung der neuen Verbindungen.

## Beispiel A

### Wirksamkeit gegen Plasmopara viticola

Blätter von Topfreben der Sorte »Müller-Thurgau« werden mit wäßriger Spritzbrühe, die 80% Wirkstoff und 20% Emulgiermittel in der Trockensubstanz enthält, besprüht.

Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewäschshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

## Beispiel B

### Wirksamkeit gegen Phytophthora infestans an Tomaten

Blätter von Topfpflanzen der Sorte »Große Fleischtomate« werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

In den Beispielen A und B zeigten insbesondere die Substanzen der Beispiele 1, 2, 4, 7, 13, 15 bzw. 32 eine bessere Wirkung als die Vergleichssubstanzen N-Trichlormethylthiophtalimid und N-Trichlormethylthiotetrahydrophthalimid.

**Patentansprüche für die Vertragsstaaten BE, CH, DE, FR, GB, IT, LI, NL, SE**

1. Oximinoessigsäureamide der Formel I

$R^1-NR^2-CO-CH=N-O-R^3$ (I),

in der

$R^1$ 2-Methyl-1-naphthyl, 2,6-Dimethyl-1-cyclohexyl oder den Rest

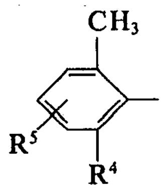

bedeutet, worin $R^4$ Wasserstoff, Methyl, Ethyl oder Chlor und $R^5$ Wasserstoff, Methyl, Phenyl, Chlor oder Brom ist,

$R^2$ den alpha-Butyro-gamma-lactonyl-, alpha-Valero-gamma-lactonyl- oder 1-Butin-3-yl-Rest oder die Gruppe $-CH(CH_3)-R^6$ bedeutet, worin $R^6$ Methoxycarbonyl oder 1,1-Dialkoxymethyl ist, und

$R^3$ einen gegebenenfalls halogensubstituierten $C_{1-6}$-Alkylrest, einen gegebenenfalls halogensubstituierten Allylrest, einen gegebenenfalls halogensubstituierten Benzylrest, oder einen Propargylrest darstellt.

2. N-(1-Methoxycarbonyl-1-ethyl)-N-(2,6-dimethylphenyl)-(O-methyl)-oximinoacetamid.
3. N-(alpha-Butyro-gamma-lactonyl)-N-(2-methyl-1-naphthyl)-(O-methyl)-oximinoacetamid.
4. N-(1-Methoxycarbonyl-1-ethyl)-N-(2-methyl-1-naphthyl)-(O-2-chlor-1-allyl)-oximinoacetamid.
5. N-(1-Methoxycarbonyl-1-ethyl)-N-(2-methyl-1-naphthyl)-(O-4-chlor-benzyl)-oximinoacetamid.
6. N-(alpha-Butyro-gamma-lactonyl)-N-(2-methyl-1-naphthyl)-(O-ethyl)-oximinoacetamid.
7. Verfahren zur Herstellung von Oximinoessigsäureamiden der Formel I gemäß Anspruch 1, dadurch gekennzeichnet, daß man

a) ein Amin der Formel II

$R^1-NH-R^2$ (II),

worin $R^1$ und $R^2$ die angegebene Bedeutung haben, mit einer Verbindung der Formel III

$R^3-O-N=CH-CO-X$ (III),

worin $R^3$ die angegebene Bedeutung hat und X ein aktiver Säurerest ist, in Gegenwart einer Base oder

b) ein Amin der Formel II mit einer Säure der Formel IV

$R^3-O-N=CH-COOH$ (IV),

worin $R^3$ die angegebene Bedeutung hat, in Gegenwart eines Säurechlorids und einer Base oder

c) eine Verbindung der Formel V

$R^1-NH-CO-CH=N-O-R^3$ (V),

worin $R^1$ und $R^3$ die angegebene Bedeutung besitzen, mit einer Verbindung der Formel

$R^2-Z$ (VI),

worin $R^2$ die angegebene Bedeutung und Z eine nucleofuge Gruppe darstellt, in Gegenwart einer Base umsetzt.

8. Fungizid, enthaltend eine Verbindung der Formel I gemäß Anspruch 1.
9. Verfahren zur Bekämpfung von Pilzen, dadurch gekennzeichnet, daß man eine fungizid wirksame Menge einer Verbindung der Formel I gemäß Anspruch 1 auf durch Pilzbefall bedrohte Flächen, Pflanzen und Saatgüter oder auf phytopathogene Pilze einwirken läßt.

**Patentansprüche für den Vertragsstaat: AT**

1. Fungizid, enthaltend ein Oximinoessigsäureamid der Formel

$R^1-NR^2-CO-CH=N-O-R^3$ (I),

in der

$R^1$ 2-Methyl-1-naphthyl, 2,6-Dimethyl-1-cyclohexyl oder den Rest

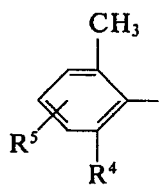

bedeutet, worin $R^4$ Wasserstoff, Methyl, Ethyl oder Chlor und $R^5$ Wasserstoff, Methyl, Phenyl, Chlor oder Brom ist,

$R^2$ den alpha-Butyro-gamma-lactonyl-, alpha-Valero-gamma-lactonyl- oder 1-Butin-3-yl-Rest oder die Gruppe $-CH(CH_3)-R^6$ bedeutet, worin $R^6$ Methoxycarbonyl oder 1,1-Dialkoxymethyl ist, und

$R^3$ einen gegebenenfalls halogensubstituierten $C_{1-6}$-Alkylrest, einen gegebenenfalls halogensubstituierten Allylrest, einen gegebenenfalls halogensubstituierten Benzylrest, oder einen Propargylrest darstellt.

2. Fungizid, enthaltend N-(1-Methoxycarbonyl-1-ethyl)-N-(2,6-dimethylphenyl)-(O-methyl)-oximinoacetamid.

3. Fungizid, enthaltend N-(alpha-Butyro-gamma-lactonyl)-N-(2-methyl-1-naphthyl)-(O-methyl)-oximinoacetamid.

4. Fungizid, enthaltend N-(1-Methoxycarbonyl-1-ethyl)-N-(2-methyl-1-naphthyl)-(O-2-chlor-1-allyl)-oximinoacetamid.

5. Fungizid, enthaltend N-(1-Methoxycarbonyl-1-ethyl)-N-(2-methyl-1-naphthyl)-(O-4-chlor-benzyl)-oximinoacetamid.

6. Fungizid, enthaltend N-(alpha-Butyro-gamma-lactonyl)-N-(2-methyl-1-naphthyl)-(O-ethyl)-oximinoacetamid.

7. Verfahren zur Herstellung von Oximinoessigsäureamiden der Formel I gemäß Anspruch 1, dadurch gekennzeichnet, daß man

a) ein Amin der Formel II

$R^1-NH-R^2$ (II),

worin $R^1$ und $R^2$ die angegebene Bedeutung haben, mit einer Verbindung der Formel III

$R^3-O-N=CH-CO-X$ (III),

worin $R^3$ die angegebene Bedeutung hat und X ein aktiver Säurerest ist, in Gegenwart einer Base oder

b) ein Amin der Formel II mit einer Säure der Formel IV

$R^3-O-N=CH-COOH$ (IV),

worin $R^3$ die angegebene Bedeutung hat, in Gegenwart eines Säurechlorids und einer Base oder

c) eine Verbindung der Formel V

$R^1-NH-CO-CH=N-O-R^3$ (V),

worin $R^1$ und $R^3$ die angegebene Bedeutung besitzen, mit einer Verbindung der Formel

$R^2-Z$ (VI),

worin $R^2$ die angegebene Bedeutung und Z eine nucleofuge Gruppe darstellt, in Gegenwart einer Base umsetzt.

8. Verfahren zur Bekämpfung von Pilzen, dadurch gekennzeichnet, daß man eine fungizid wirksame Menge einer Verbindung der Formel I gemäß Anspruch 1 auf durch Pilzbefall bedrohte Flächen, Pflanzen und Saatgüter oder auf phytopathogene Pilze einwirken läßt.

**Claims for the contracting states: BE, CH, DE, FR, GB, IT, LI, NL, SE**

1. An oximinoacetamide of the formula I

$$R^1-NR^2-CO-CH=N-O-R^3 \quad (I),$$

where

$R^1$ is 2-methyl-1-naphthyl, 2,6-dimethyl-1-cyclohexyl or

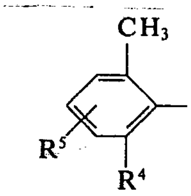

where $R^4$ is hydrogen, methyl, ethyl or chlorine, and $R^5$ is hydrogen, methyl, phenyl, chlorine or bromine,

$R^2$ is $\alpha$-butyro-$\gamma$-lactonyl, $\alpha$-valero-$\gamma$-lactonyl, 1-butyn-3-yl or $-CH(CH_3)-R^6$, where $R^6$ is methoxycarbonyl or 1,1-dialkoxymethyl, and

$R^3$ is an optionally halogen-substituted alkyl of 1 to 6 carbon atoms, and optionally halogen-substituted allyl, an optionally halogen-substituted benzyl, or propargyl.

2. N-(1-Methoxycarbonyl-1-ethyl)-N-(2,6-dimethylphenyl)-(O-methyl)-oximinoacetamide.
3. N-($\alpha$-Butyro-$\gamma$-lactonyl)-N-(2-methyl-1-naphthyl)-(O-methyl)-oximinoacetamide.
4. N-(1-Methoxycarbonyl-1-ethyl)-N-(2-methyl-1-naphthyl)-(O-chloro-1-allyl)-oximinoacetamide.
5. N-(1-Methoxycarbonyl-1-ethyl)-N-(2-methyl-1-naphthyl)-(O-4-chlorobenzyl)-oximinoacetamide.
6. N-($\alpha$-Butyro-$\gamma$-lactonyl)-N-(2-methyl-1-naphthyl)-(O-ethyl)-oximinoacetamide.
7. A process for the preparation of an oximinoacetamide of the formula I as claimed in claim 1, wherein

(a) an amine of the formula II

$$R^1-NH-R^2 \quad (II),$$

where $R^1$ and $R^2$ have the said meanings, is reacted with a compound of the formula III

$$R^3-O-N=CH-CO-X \quad (III),$$

where $R^3$ has the said meanings and X is an active acid radical, in the presence of a base, or

(b) an amine of the formula II is reacted with an acid of the formula IV

$$R^3-O-N=CH-COOH \quad (IV),$$

where $R^3$ has the said meanings, in the presence of an acid chloride and a base, or

(c) a compound of the formula V

$$R^1-NH-CO-CH=N-O-R^3 \quad (V),$$

where $R^1$ and $R^3$ have the said meanings, is reacted with a compound of the formula VI

$$R^2-Z \quad (VI),$$

where $R^2$ has the said meanings and Z is a nucelofugic group, in the presence of a base.

8. A fungicide containing a compound of the formula I as claimed in claim 1.
9. A process for combating fungi, wherein, a fungicidally effective amount of a compound of the formula I as claimed in claim 1 is allowed to act on the areas, plants or seed threatened by fungal attack, or on phytopathogenic fungi.

**Claims for the contracting state: AT**

1. A fungicide containing an oximinoacetamide of the formula I

$$R^1-NR^2-CO-CH=N-O-R^3 \quad (I),$$

where

$R^1$ is 2-methyl-1-naphthyl, 2,6-dimethyl-1-cyclohexyl or

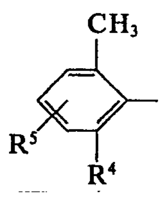

where $R^4$ is hydrogen, methyl, ethyl or chlorine, and $R^5$ is hydrogen, methyl, phenyl, chlorine or bromine,

$R^2$ is $\alpha$-butyro-$\gamma$-lactonyl, $\alpha$-valero-$\gamma$-lactonyl, 1-butyn-3-yl or $-CH(CH_3)-R^6$, where $R^6$ is methoxycarbonyl or 1,1-dialkoxymethyl, and

$R^3$ is an optionally halogen-substituted alkyl of 1 to 6 carbon atoms, and optionally halogen-substituted allyl, an optionally halogen-substituted benzyl, or propargyl.

2. A fungicide containing N-(1-methoxycarbonyl-1-ethyl)-N-(2,6-dimethylphenyl)-(O-methyl)-oximinoacetamide.

3. A fungicide containing N-($\alpha$-butyro-$\gamma$-lactonyl)-N-(2-methyl-1-naphthyl)-(O-methyl)-oximinoacetamide.

4. A fungicide containing N-(1-methoxycarbonyl-1-ethyl)-N-(2-methyl-1-naphthyl)-(O-2-chloro-1-allyl)-oximinoacetamide.

5. A fungicide containing N-(1-methoxycarbonyl-1-ethyl)-N-(2-methyl-1-naphthyl)-(O-4-chlorobenzyl)-oximinoacetamide.

6. A fungicide containing N-($\alpha$-butyro-$\gamma$-lactonyl)-N-(2-methyl-1-naphthyl)-(O-ethyl)-oximinoacetamide.

7. A process for the preparation of an oximinoacetamide of the formula I as claimed in claim 1, wherein

(a) an amine of the formula II

$$R^1-NH-R^2 \quad (II),$$

where $R^1$ and $R^2$ have the said meanings, is reacted with a compound of the formula III

$$R^3-O-N=CH-CO-X \quad (III),$$

where $R^3$ has the said meanings and X is an active acid radical, in the presence of a base, or

(b) an amine of the formula II is reacted with an acid of the formula IV

$$R^3-O-N=CH-COOH \quad (IV),$$

where $R^3$ has the said meanings, in the presence of an acid chloride and a base, or

(c) a compound of the formula V

$$R^1-NH-CO-CH=N-O-R^3 \quad (V),$$

where $R^1$ and $R^3$ habe the said meanings, is reacted with a compound of the formula VI

$$R^2-Z \quad (VI),$$

where $R^2$ has the said meanings and Z is a nucelofugic group, in the presence of a base.

8. A process for combating fungi, wherein, a fungicidally effective amount of a compound of the formula I, as defined in claim 1 is allowed to act on the areas, plants or seed threatened by fungal attack, or on phytopathogenic fungi.

**Revendications pour les Etats contractants: BE, CH, DE, FR, GB, IT, LI, NL, SE**

1. Oximino-acétamides de la formule I

$$R^1-NR^2-CO-CH=N-O-R^3 \quad (I),$$

dans laquelle

$R^1$ désigne un groupe 2-méthyl-1-naphthyle ou 2,6-diméthyl-1-cyclohexyle ou un groupe de la formule

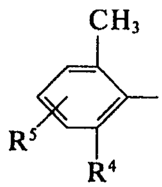

dans laquelle $R^4$ peut être un atome d'hydrogène ou de chlore ou un radical méthyle ou éthyle et $R^5$ un atome d'hydrogène, de chlore ou de brome ou un groupe méthyle ou phényle,

$R^2$ représente un groupe alpha-butyro-gamma-lactonyle, alpha-valéro-gamma-lactonyle ou 1-butyne-3-yle ou un groupe $-CH(CH_3)-R^6$, où $R^6$ est un groupe méthoxycarbonyle ou 1,1-dialcoxyméthyle et

$R^3$ désigne un radical alkyle en $C^1$ à $C^6$ éventuellement halo-substitué, un groupe allyle éventuellement halo-substitué, un groupe benzyle éventuellement halo-substitué ou un groupe propargyle.

2. Le N-(1-méthoxycarbonyl-1-éthyl)-N-(2,6-diméthylphényl)-(O-méthyl)-oximino-acétamide.
3. Le N-(alpha-butyro-gamma-lactonyl)-N-(2-méthyl-1-naphthyl)-(O-méthyl)-oximino-acétamide.
4. Le N-(1-méthoxycarbonyl-1-éthyl)-N-(2-méthyl-1-naphtyl)-(O-2-chloro-1-allyl)-oximino-acétamide.
5. Le N-(1-méthoxycarbonyl-1-éthyl)-N-(2-méthyl-1-naphtyl)-(O-4-chloro-benzyl)-oximino-acétamide.
6. Le N-(alpha-butyro-gamma-lactonyl)-N-(2-méthyl-1-naphthyl)-(O-éthyl)-oximino-acétamide.
7. Procédé de préparation d'oximino-acétamides de la formule I selon la revendication 1, caractérisé en ce que l'on fait réagir:

soit a) une amine de la formule II

$$R^1-NH-R^2 \quad (II),$$

dans laquelle $R^1$ et $R^2$ possèdent la signification définie, en présence d'une base avec un composé de la formule III

$$R^3-O-N=CH-CO-X \quad (III),$$

dans laquelle $R^3$ possède la signification définie et X est un reste acide réactif;

soit b) une amine de la formule II, en présence d'une base et d'un chlorure d'acide, avec un acide de la formule IV

$$R^3-O-N=CH-COOH \quad (IV),$$

dans laquelle $R^3$ possède la signification définie,

ou c) un composé de la formule V

$$R^1-NH-CO-CH=N-O-R^3 \quad (V),$$

dans laquelle $R^1$ et $R^3$ possèdent la signification définie, en présence d'une base avec un composé de la formule

$$R^2-Z \quad (VI),$$

dans laquelle $R^2$ possède la signification définie et Z est un groupe nucléofuge.

8. Composition fongicide, contenant un composé de la formule I selon la revendication 1.

9. Procédé de lutte contre les champignons, caractérisé en ce que l'on fait agir une quantité efficace du point de vue fongicide d'un composé de la formule I selon la revendication 1 sur les surfaces, plantes ou semences à protéger des champignons ou sur les champignons phytopathogènes.

**Revendications pour l'Etats contractant: AT**

1. Composition fongicide, contenant un oximino-acétamide de la formule

$$R^1-NR^2-CO-CH=N-O-R^3 \quad (I),$$

dans laquelle

$R^1$ désigne un groupe 2-méthyl-1-naphthyle ou 2,6-diméthyl-1-cyclohexyle ou un groupe de la formule

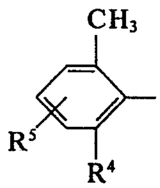

dans laquelle $R^4$ peut être un atome d'hydrogène ou de chlore ou un radical méthyle ou éthyle et $R^5$ un atome d'hydrogène, de chlore ou de brome ou un groupe méthyle ou phényle,

$R^2$ représente un groupe alpha-butyro-gamma-lactonyle, alpha-valéro-gamma-lactonyle ou 1-butyne-3-yle ou un groupe $-CH(CH_3)-R^6$, où $R^6$ est un groupe méthoxycarbonyle ou 1,1-dialcoxyméthyle et

$R^3$ désigne un radical alkyle en $C^1$ à $C^6$ éventuellement halo-substitué, un groupe allyle éventuellement halo-substitué, un groupe benzyle éventuellement halo-substitué ou un groupe propargyle.

2. Composition fongicide, contenant du N-(1-méthoxycarbonyl-1-éthyl)-N-(2,6-diméthyl-phényl)-(O-méthyl)-oximino-acétamide.

3. Composition fongicide, contenant du N-(alpha-butyro-gamma-lactonyl)-N-(2-méthyl-1-naphthyl)-(O-méthyl)-oximino-acétamide.

4. Composition fongicide, contenant du N-(1-méthoxycarbonyl-1-éthyl)-N-(2-méthyl-1-naphtyl)-(O--2-chloro-1-allyl)-oximino-acétamide.

5. Composition fongicide, contenant du N-(1-méthoxycarbonyl-1-éthyl)-N-(2-méthyl-1-naphtyl)-(O-éthyl)-oximino-acétamide.

6. Composition fongicide, contenant du N-(alpha-butyro-gamma-lactonyl)-N-(2-méthyl-1-naphthyl)-(O-éthyl)-oximino-acétamide.

7. Procédé de préparation d'oximino-acétamides de la formule I selon la revendication 1, caractérisé en ce que l'on fait réagir:

soit a) une amine de la formule II

$$R^1-NH-R^2 \quad (II),$$

dans laquelle $R^1$ et $R^2$ possèdent la signification définie, en présence d'une base avec un composé de la formule III

$$R^3-O-N=CH-CO-X \quad (III),$$

dans laquelle $R^3$ possède la signification définie et X est un reste acide réactif;

soit b) une amine de la formule II, en présence d'une base et d'un chlorure d'acide, avec un acide de la formule IV

$$R^3-O-N=CH-COOH \quad (IV),$$

dans laquelle $R^3$ possède la signification définie,

ou c) un composé de la formule V

$$R^1-NH-CO-CH=N-O-R^3 \quad (V),$$

dans laquelle $R^1$ et $R^3$ possèdent la signification définie, en présence d'une base avec un composé de la formule

$R^2$—Z (VI),

dans laquelle $R^2$ possède la signification définie et Z est un groupe nucléofuge.

8. Procédé de lutte contre les champignons, caractérisé en ce que l'on fait agir une quantité efficace du point de vue fongicide d'une composé de la formule I selon la revendication 1 sur les surfaces, plantes et semences à protéger des champignons ou sur les champignons phytopathogènes.